# EUROPEAN PATENT APPLICATION

(11) **EP 0 640 351 A1**
(43) Date of publication of application: **01.03.1995**
(21) Application number: 94201925.8
(22) Date of filing: 05.07.1994
(51) Int. Cl.: A61L 2/00, C07K 1/14, G01N 33/569

(54) **Method for the separtion of bacterial lipopolysaccharides from protein-containing solutions**

(30) Priority: 26.07.1993 EP 93202201
(71) Applicant: DUPHAR INTERNATIONAL RESEARCH B.V, NL-1381 CP Weesp (NL)
(72) Inventor: Kompier, Ronald, NL-1380 AC Weesp (NL); Sloothaak, Johannes B., NL-1380 AC Weesp (NL); van Scharrenburg, Gustaaf J.M., NL-1380 AC Weesp (NL); Bredschneijder, Boudewijn C., NL-1380 AC Weesp (NL); Brands, Rudi, NL-1380 AC Weesp (NL)
(74) Representative: Swaters, Pieter D.

(57) **Abstract**

The present invention is concerned with a method for the separation of bacterial lipopolysaccharides (LPS) from a protein-containing solution wherein the LPS are set free into the solution by using at least one suitable solubilizing agent, and wherein the solution is brought into contact with a glass surface, whereafter the LPS-loaded glass surface is separated from the protein-containing solution.
Furthermore, LPS from a solution can easily be detected according to the present invention by conventional methods, by first binding said LPS to a glass surface in the above described way.

## Description

The present invention is concerned with a method for the separation of bacterial lipopolysaccharides from a protein-containing solution, and with a method for the detection of lipopolysaccharides.

It is well known that bacterial lipopolysaccharides (LPS), known as endotoxins, can cause (severe) pyrogenic reactions upon administration to animals and humans. Thus, the LPS-mediated biological effects of shock, fever, leucopenia, hyperglycemia, intravascular coagulation and others have been recognized. For this reason the occurence of LPS above a certain threshold concentration will withhold allowance of pharmaceuticals or biologicals for administration to animals and humans.

In solution LPS are arranged in a bilayer similar to a bilayer of phospholipids. Bivalent cations, e.g. Ca²⁺, stabilize this bilayer. These huge bilayer structures can be reduced in size by the use of detergents or bile salts. The thus formed mixed micelles of LPS and detergents or bile salts have smaller molecular weights which offer an opportunity to separate the micelle-bound LPS from product by ultrafiltration or microfiltration methods.

Making use of the basic characteristics of bacterial LPS, also several other methods have been described claiming removal of LPS from the product of interest. Amongst these is the application of organic substances such as phenol, trichloroacetic acid, or a phenol/chloroform/petroleum-ether mixture, or the neutralization of LPS by endotoxin binding proteins (ENP). This latter method is suited for removal at laboratory scale, but gives significant protein losses. Furthermore, LPS have been removed by making use of resins, charged filters, barium sulfate, detergents and bile acids or derivatives thereof (see e.g. U.S. patent 4315919 and Sweadner et al., Appl. and Env. Microbiology 34, 1977, 382-385), either or not in combination with chelators.

Although the above methods have resulted in clearance of LPS from protein-containing solutions to various extends at a laboratory scale, neither of these methods, however, is suited to separate LPS from such solutions in a cost effective and efficient manner on a industrial scale.

The purpose of the present invention is to provide a simple and reliable method for the removal of LPS from a protein-containing solution. This method can well be used on an industrial scale.

For several purposes there is a need for a diagnostic method for quantitative or qualitative determination of bacterial LPS in solution (LAL tests). Classical tests for this purpose are the gel clot test and the related chromogenic test. However to be accurate, both of these tests place great demands with respect to the reaction conditions. Results, therefore, are often difficult to interpret, especially when the LPS levels are in the low ranges of detection. Therefore, another purpose of the present invention is to provide a simple and reliable method for the detection and/or quantitation of LPS in solutions, in particular solutions containing interfering compounds.

Surprisingly, it has been found that LPS can be separated efficiently from a protein-containing solution by adding at least one suitable solubilizing agent to this solution and bringing this solution into contact with a glass surface, followed by separation of the LPS-loaded glass surface from the protein-containing solution. The method according to this invention enables substantial removal of LPS from solutions containing water-soluble as well as membrane-bound proteins. The method of the present invention has proved to be particularly suitable for the removal of LPS from heavily LPS-contaminated protein-containing solutions. The method of the invention is so simple, that it can easily be applied on an industrial scale.

Suitable solubilizing agents to be used in the method of the invention are detergents. Such detergents comprise both non-ionic and ionic surface active substances. Non-ionic surface active substances also include bile acid salts, such as cholates, ionic surface active substances also cover substances with a dipolar ionic structure. The solubilizing agents to be chosen should not affect the desired biological activity of the protein-containing solution.

Glass surfaces should be understood to also include vitreous surfaces, i.e. surfaces from vitreous materials such as ceramic materials. Glass, however, in particular normal non-pretreated glass, is preferred. Suitable examples of glass surfaces to be used in the method of the invention are glass beads, glass vials, glass tubes, glass plates and glass filters.

The method according to the present invention is preferably performed under certain favourable conditions, dependent on the composition of the protein-containing solution and on the character of the ingredients thereof, in particular the protein or proteins and/or the contaminating LPS. The conditions should be chosen in such a manner, that the desired biological activity of the protein-cotaining solution is not affected. Preferably a substantially aqueous solution is used with a low ionic strength. The temperature of the solution during the treatment is not very critical, but generally about room temperature is well suited. Preferably the solution has a sufficiently high pH, for example in the range of approx. 8 - 9.5. The pH can be adjusted by adding a suitable buffer, such as a phosphate buffer, HEPES buffer or TRIS buffer. Although the solution may be treated with the glass surface (incubated) overnight, an incubation time of less then one hour, even of approx. 10 minutes, is already sufficient generally.

In addition, according to another aspect of the present invention, the amount of LPS separated from a solution by binding to a glass surface can be detected and determinated accurately, e.g. by commercial gel clot test or chromogenic test kits under reaction conditions recommended by the suppliers of the kits.

The method according to the present invention is, therefore, of wide use, ranging from laboratory applications to the production of LPS-free biologicals. For example, proteins derived from animals (e.g. albumin), often found to be LPS-contaminated, can be purified prior to further use, e.g. as a tissue culture constituent. Monoclonal antibodies, therapeutic proteins, enzymes, both viral and bacterial vaccines, and biological response modifiers (growth factors, etc.) destined for clinical or laboratory purposes thus are purified from LPS contaminations, e.g. derived from tissue culture media or bacteria cell walls, or arised from post culture processing. For instance, egg-derived vaccines, such as inactivated vaccines, can easily be decontaminated.

The method according to the invention may be further explained as follows. First, the LPS is set free and becomes part of small micelles by addition of at least one solubilizing agent, generally a detergent, or by applying several solubilizing agents, e.g. by combinatory use of detergent and a chelator, preferably under the favourable conditions as described above. Suitable metal-ion chelators are poly(acetoxylated amines), such as EDTA, DTPA, etc., and optionally hydroxylated polycarboxylic acids, such as citric acid, oxalic acid, tartaric acid, etc., as well as their alkali metal salts. Preferably a detergent is used which forms small micelles and thus can easily be removed from the substantially aqueous solution, such as cholates ( e.g. deoxycholate (DOC) or analogues thereof). Thereupon, the LPS are removed from the bulk solution by adsorption to a suitable glass surface and the LPS-loaded glass surface is subsequently removed. As a next step, the detergent can be removed, e.g. by (dia)filtration or binding to a resin (e.g. Amberlite^{®}). The resulting protein solution is almost completely freed from LPS.

It has been found that glass surfaces, e.g. in the form of glass beads (as an example, having a diameter of 5 mm and a surface of 74.2 mm²) bind at least 10µg LPS or ≧ 10⁵ EU (= endotoxin unit as measured with the LAL test; 1 ng of LPS = approx. 10 EU). The detergent DOC can easily be removed by ultrafiltration using a membrane having a cut-off value of at least 30 or 50 Kd. Protein recovery ≧ 95% is possible if suitable well-known methods and materials for removal of detergent are applied. Convincing data are obtained with the endotoxin removal from a haemagglutinin containing solution: see the Examples. Recovery of the viral membrane protein is 100% as measured by protein determination and immunochemical determination by Single Radial Diffusion method before removal of the detergent which indicates that no loss of protein due to absorption to the glass can be detected.

The invention is illustrated in greater detail by the following specific Examples.

### EXAMPLES

### EXAMPLE I

In the Examples the following **GENERAL PROCEDURE** is used.
For the preparation of a phosphate buffer solution the following substances are mixed and dissolved in water: EDTA.2Na (1860 mgram/l), NaCl (877.5 mgram/l), Na₂HPO₄ (710 mgram/l). The pH is adjusted at 9.5 by the addition of 0.5 mol/l of NaOH.
A deoxycholate (DOC) stock solution is prepared by adjusting a 10% v/v solution of DOC in water at a pH of 9.5 in the same above manner. Other detergent stock solutions are prepared in a corresponding manner.
Starting solutions containing protein, e.g. Influenza hemagglutinin, heavily contaminated with bacterial LPS (e.g. approx. 300,000 EU/ml) are diluted with 9 volumes of the above buffer solution. The protein concentration obtained is 125 µg/ml.
DOC stock solution is added to a final concentration of 1% and the mixture is incubated for at least one hour at 20°C.
All experiments are carried out in stainless steel tubes.
Next, glass beads (diameter 5 mm, surface 74.2 mm₂) are added. The mixture is swirled gently overnight at 20°C, after which the amount of free endotoxins, not bound to the glass beads, is measured by using the LAL test method. The minimal amount of glass to be added can be calculated from the original LPS content and the information given above. Excess of glass does not influence the LPS removal.
The almost LPS-free solution (e.g. 0.5-50 EU/ml) of the protein can be obtained by transfer of the solution to another container and thus separating the solution from the glass beads. The amount of glass-bound LPS can be detected by the chromogenic test method.
Under the above conditions of a low ionic strength and a pH above 7, the DOC can easily be removed by ultrafiltration, e.g. by using a filter of a suitable molecular weight cut off (MWCO). It will be clear, that the choice of the filter used largely depends on the size of the proteins of interest and of the detergent micelles. As an example, diafiltration using a membrane with a MWCO of 50 Kd against the desired buffer allows for efficient DOC removal from an Influenza hemagglutinin preparation.
Alternatively, DOC can be bound to a suitable resin (eg. Amberlite XAD-4).

### Use of various detergents.

Solutions containing Influenza hemagglutinin (HA-protein), heavily contaminated with endotoxins (from the outer membrane of E. coli 0111 /B4) are treated under the conditions of the above general procedure with 2 glass beads in the presence of various detergents. The applied detergents are: deoxycholate (DOC), N-octylglucoside (N-oct.-gl.), cetyltrimethylammoniumbromide (CTAB), and Triton^{®} X100. The following results are obtained:

| detergent | conc. | bacterial LPS |
|---|---|---|
| ---- | 0% | 96,000 EU/ml |
| N-oct.-gl. | 1% | < 3,000 EU/ml |
| CTAB | 1% | < 1,500 EU/ml |
| Triton X 100 | 1% | < 1,500 EU/ml |
| DOC | 1% | < 50 EU/ml |

### EXAMPLE II

### Variation in detergent concentration.

Under the same conditions as described in Example I various detergent concentrations are tested; HA-protein is used in this Example. The following results are obtained:

| detergent | conc. | bacterial LPS |
|---|---|---|
| ---- | 0% | 96,000 EU/ml |
| DOC | 0.5% | 3,000 EU/ml |
| DOC | 1 % | < 300 EU/ml |

### EXAMPLE III

### Variation in proteins.

Under the same conditions as described in Example I, the method of the invention is used for the decontamination of various proteins. The following proteins are used: hemagglutinin (HA), bovine serum albumin (BSA), and rabbit immunoglobulin (IgG). The following results are obtained:

| detergent | conc. | protein | bacterial LPS |
|---|---|---|---|
| ---- | 0% | HA + BSA + IgG | 6,000 EU/ml |
| DOC | 1 % | HA | < 50 EU/ml |
| DOC | 1 % | BSA | < 300 EU/ml |
| DOC | 1% | IgG | < 1,500 EU/ml |

### EXAMPLE IV

### Variation in endotoxin contamination

Under the same conditions as described in Example I, HA-protein, contaminated with various endotoxins, is investigated in the method of the invention, viz.: E. coli 0111:B4, Salmonella minnesota Re 595 (Re mutant), and Pseudomonas aeruginosa 10. The following results are obtained:

| detergent | conc. | endotoxin (10⁵ EU/ml) | bacterial LPS |
|---|---|---|---|
| --- | 0% | E. coli | 122,880 EU/ml |
| DOC | 1% | E. coli | 524 EU/ml |
| --- | 0% | Salmonella | 3,840 EU/ml |
| DOC | 1% | Salmonella | 960 EU/ml |
| --- | 0% | Pseudomonas | 30,720 EU/ml |
| DOC | 1 % | Pseudomonas | 240 EU/ml |

### EXAMPLE V

### Presence or absence of glass surface.

4 ml of a solution of heavily endoxin-contaminated HA-protein (125 µg/ml), 0% or 1.1% DOC, and phosphate buffer, is treated under the same conditions as described in Example I, in the presence or absence of glass beads. The following results are obtained:

| DOC | glass beads | endotoxins (E. coli) |
|---|---|---|
| 0% | 0 | 182,000 EU/ml |
| 1.1% | 0 | 182,000 EU/ml |
| 1.1% | 2 | < 50 EU/ml |

Corresponding experiments are carried out with HA protein contaminated with Salmonella minnesota, giving the following results:

| DOC | glass beads | endotoxins (Salm. minn.) |
|---|---|---|
| 1% | 0 | 7,680 EU/ml |
| 1% | 2 | 960 EU/ml |

## Claims

1. Method for the separation of bacterial lipopolysaccharides (LPS) from a protein-containing solution wherein the LPS are set free into the solution by using at least one suitable solubilizing agent, characterized in that the solution is brought into contact with a glass surface, whereafter the LPS-loaded glass surface is separated from the protein-containing solution.

2. Method according to claim 1, characterized in that the LPS are set free by the addition of a suitable detergent.

3. Method according to claim 1, characterized in that the LPS are set free by the combined addition of a suitable detergent and a metal-ion chelator.

4. Method according to claim 2 or 3, characterized in that as a detergent desoxycholate or an analogue thereof is used.

5. Method according to claim 2, 3 or 4, characterized in that the method is performed in a substantially aqueous solution with a low ionic strength, and at a pH in the range of approx. 8 -9.5.

6. Method according to any of claims 1-5, characterized in that the agent setting the LPS free into the solution is removed from the protein-containing solution after removal of the LPS-loaded glass surface.

7. Method for the determination of bacterial LPS in solutions, characterized in that the LPS are bound to a glass surface followed by separation of the LPS-loaded glass surface, whereafter the amount of LPS bound to the glass surface is determined by methods known per se.
